# EUROPEAN PATENT APPLICATION

(11) **EP 1 338 279 A1**
(43) Date of publication of application: **27.08.2003**
(21) Application number: 01982746.8
(22) Date of filing: 08.11.2001
(51) Int. Cl.: A61K 31/202, A61K 9/08, A61P 27/02

(54) **REMEDIES FOR KERATOCONJUNCTIVAL DISEASES CONTAINING FARNESYL ACETATE AS THE ACTIVE INGREDIENT**

(30) Priority: 08.11.2000 JP 2000340115
(71) Applicant: SANTEN PHARMACEUTICAL CO., LTD., Higashiyodogawa-ku, Osaka-shi, Osaka 533-8651 (JP)
(72) Inventor: NAKATA, Katsuhiko, c/o SANTEN PHARMA. CO., LTD., Ikoma-shi, Nara 630-0101 (JP); NAKAMURA, Masatsugu, c/o SANTEN PHARMA. CO., LTD., Ikoma-shi, Nara 630-0101 (JP); ENDO, Ken-ichi, c/o SANTEN PHARMA. CO., LTD., Ikoma-shi, Nara 630-0101 (JP); KUWANO, Mitsuaki, c/o SANTEN PHARMA. CO., LTD., Ikoma-shi, Nara 630-0101 (JP); IBUKI, Hajime, c/o SANTEN PHARMACEUTICAL CO., LTD., Ikoma-shi, Nara 630-0101 (JP)
(74) Representative: Peaucelle, Chantal
(86) International application number: JP0109760
(87) International publication number: WO02038149

(57) **Abstract**

The present invention provides new substances which promote production and secretion of mucin in ophthalmic tissues. Therapeutic agents for keratoconjunctival diseases in the present invention comprise farnesylacetic acid or a salt thereof as an active ingredient. These therapeutic agents for the keratoconjunctival diseases can be applied to dry eye, keratitis, conjunctivitis, corneal erosion, corneal ulcer and the like.

## Description

### Technical Field

The present invention relates to therapeutic agents for keratoconjunctival diseases comprising farnesylacetic acid or a salt thereof as an active ingredient.

### Background Art

Organisms are equipped with a defense mechanism for protecting a mucous membrane. Epithelial cells of the mucous membrane are always covered with a viscous exocrine liquid containing mucin secreted from exocrine gland. In eyes, tear plays such a role. Tear layer consists of three layers, which are lipid layer, aqueous layer and mucin layer, and keratoconjunctiva epithelial cells are in contact with the mucin layer. Mucin, which constitutes the mucin layer, is a glycoprotein secreted from goblet cells of conjunctiva and the keratoconjunctiva epithelial cells, makes the tear hold well and keeps wetness of cornea and conjunctiva.

Dry eye is a disease which indicates symptoms of eye drying and causes a corneal epithelial disorder owing to abnormalities of tear fluid. Examples of symptoms of dry eye are symptom of eye drying, corneal hyperemia, itching, a sense of a foreign body, etc. When dry eye becomes serious, it sometimes causes visual loss. A decrease of tear secretion, instability of the tear layer and the like are considered as causes of incidence of dry eye, but these causes have not been clarified yet.

Accordingly, if there is any substance having an action of promoting the secretion of mucin into tear fluid, it is expected that such a substance is useful to treat keratoconjunctival diseases having a trouble on keratoconjunctiva epithelium such as dry eye, keratitis, conjunctivitis, corneal erosion and corneal ulcer.

Japanese Laid-open Patent Publication No. 194363/1997 discloses that gefarnate, which is an ester of farnesylacetic acid with geraniol, has a promotive action on production and secretion of mucin in ophthalmic tissues and is effective for the keratoconjunctival diseases such as dry eye.

However, there has been no report concerning application of farnesylacetic acid, which is a free carboxylic acid of gefarnate, to an ophthalmological field, and it is not known how farnesylacetic acid acts on the keratoconjunctival diseases at all.

Accordingly, it was a very interesting subject to find pharmacological effects of farnesylacetic acid on the keratoconjunctival diseases.

### Disclosure of the Invention

Studying a promotive action of farnesylacetic acid on production and secretion of mucin in order to find its new pharmacological actions, the present inventors found that farnesylacetic acid has a remarkable promotive action on production and secretion of mucin. It turned out that farnesylacetic acid has an effect equal to that of gefarnate even at a much lower concentration than that of gefarnate.

The present invention relates to therapeutic agents for keratoconjunctival diseases such as dry eye, keratitis, conjunctivitis, corneal erosion and corneal ulcer comprising farnesylacetic acid represented by the following formula or a salt thereof as an active ingredient, and their effects will be described in detail in the later section of Pharmacological tests.

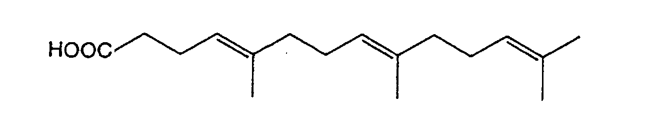

Salts in the present compound can be any salts which are pharmaceutically acceptable, and examples thereof are salts with an alkali metal or an alkaline earth metal such as sodium, potassium or calcium, an ammonium salt and salts with an organic amine such as diethylamine or triethanolamine.

Examples of dosage forms of farnesylacetic acid or the salt thereof are ophthalmic preparations such as ophthalmic solutions and eye ointments and injections, and farnesylacetic acid or the salt thereof can be formulated in such preparations utilizing the widely-used method. Ophthalmic solutions, for example, can be prepared using optionally isotonic agents such as sodium chloride and concentrated glycerol; buffers such as sodium phosphate and sodium acetate; surfactants such as Polysolvate 80, polyoxyethylene hydrogenated castor oil 60 and macrogol 4000; stabilizers such as sodium citrate and disodium edetate; preservatives such as benzalkonium chloride and paraben; and pH adjustors such as sodium hydroxide and hydrochloric acid. It is desirable to adjust pH to around neutrality, and it is desirable to adjust an osmotic pressure ratio to about 1.0.

The present invention also relates to a method of treating the keratoconjunctival diseases comprising administering to a patient an effective amount of farnesylacetic acid or the salt thereof, and a method of promoting the production of mucin or a method of treating diseases concerning the production of mucin comprising administering to a patient an effective amount of farnesylacetic acid or the salt thereof.

The dose can be appropriately selected depending upon symptoms, age, dosage form, etc. In the case of ophthalmic solutions, a solution of 0.0001 to 3% (w/v), preferably 0.001 to 1% (w/v), can be instilled once to several times per day.

### Best Mode for Carrying out the Invention

Pharmacological tests: An effect of farnesylacetic acid on dry eye was studied by using lacrimal gland-nictitating membrane-harderian gland-enucleated rabbit dry eye models.

### Preparation of rabbit dry eye models

Lacrimal gland-nictitating membrane-harderian gland-enucleated rabbit dry eye models were prepared by reference to the method of Girbard et al. (Invest. Ophthalmol. Vis. Sci., 28: 225-228 (1987)). Namely, a 7:1 mixture of a 5% ketamine hydrochloride injection and a 2% xylazine hydrochloride injection was injected into rabbits in an amount of 1 ml/kg to anesthetize the rabbits systemically. A 0.4% oxybuprocaine hydrochloride ophthalmic solution was instilled into the rabbits, lower lid skin was dissected in length of about 5 mm along an orbital bone, and then a lacrimal gland was enucleated from the outside of a conjunctival sac. The skin at the dissected site was sutured with 4-0 sutures, and an ofloxacin eye ointment was applied to the sutured site. Next, a harderian gland was enucleated while drawing it out with a nictitating membrane. Finally, the ofloxacin eye ointment was applied to the enucleated site.

### Preparation of ophthalmic solutions

Farnesylacetic acid ophthalmic solutions were prepared by diluting farnesylacetic acid with the following vehicle so that its concentrations were 0.75, 7.5 and 75 µM. A gefarnate ophthalmic solution was prepared by diluting gefarnate with the same vehicle so that its concentration was 7.5 mM. All the obtained ophthalmic solutions had pH of 7.4 and osmotic pressure of 1.0.

| Vehicle (in 100 ml) | |
|---|---|
| Polyoxyethylene hydrogenated castor oil 40 | 0.3 g |
| NaH₂PO₄•2H₂O | 1.0 g |
| Disodium edetate | 0.05 g |
| NaCl | 0.4 g |
| Sterile purified water | q.s. |

### Administration

Each farnesylacetic acid ophthalmic solution (50 µ l) was instilled into the lacrimal gland-nictitating membrane-harderian gland-enucleated rabbits six times per day for two weeks (four rabbits and eight eyes per group. The vehicle and gefarnate were instilled similarly as a control group and a comparative group respectively.

### Method of evaluation

Effects on dry eye were evaluated by measuring rose bengal intake. A 7:1 mixture of a 5% ketamine hydrochloride injection and a 2% xylazine hydrochloride injection was injected into the rabbits in an amount of 1 ml/kg to anesthetize the rabbits systemically, then eyelids were opened with an eye speculum, and the conjunctival sacs were filled with physiological saline containing 1% rose bengal. The eyes were maintained for 10 seconds and then washed with physiological saline. A 5% pentobarbital injection was injected into the rabbits to euthanatize them, and eyeballs were enucleated. Corneal pieces were prepared with a trephine having an inner diameter of 8 mm, put into 1 ml of 2 M sodium hydroxide and allowed to stand under light resistance for one day to dissolve corneal tissues completely. Absorbance at a wavelength of 560 nm of the obtained tissue-dissolved solutions was measured with a spectrophotometer.

Rose bengal stains a portion containing no mucin layer in keratoconjunctiva. If a mucin layer exists, rose bengal is prevented from permeating into keratoconjunctival tissues, but if the mucin layer does not exist, rose bengal can easily reach the keratoconjunctival tissues. Accordingly, when the mucin layer decreases because of dry eye, rose bengal intake of the corneal tissues increases, and the absorbance of the tissue-dissolved solution increases.

### Results

Measured results of the absorbance are shown in Table 1. Rose bengal intake in farnesylacetic acid administration groups was apparently less than that in the vehicle administration group, and the rose bengal intake decreased approximately concentration-dependently in the farnesylacetic acid administration groups. Further, it turned out that farnesylacetic acid (75 µ M administration group) exhibits an effect equal to that of gefarnate (7.5 mM administration group) even at a concentration which is about 1/100 as high as that of gefarnate. From the above-mentioned results, since farnesylacetic acid promotes production and secretion of mucin, farnesylacetic acid is expected to be useful for keratoconjunctival diseases such as dry eye.

| | Absorbance |
|---|---|
| Control group (vehicle) | 0.236 |
| Farnesylacetic acid 0.75 µM | 0.169 |
| 7.5 µM | 0.139 |
| 75 µM | 0.093 |
| Comparative group (gefarnate 7.5 mM) | 0.075 |

### Industrial Applicability

The above-mentioned results of the pharmacological tests explicitly show that farnesylacetic acid has a promotive effect on production and secretion of mucin and is useful as therapeutic agents for keratoconjunctival diseases such as dry eye, keratitis, conjunctivitis, corneal erosion and corneal ulcer.

## Claims

1. A therapeutic agent for a keratoconjunctival disease comprising farnesylacetic acid or a salt thereof as an active ingredient.

2. The therapeutic agent for the keratoconjunctival disease as claimed in claim 1, wherein the keratoconjunctival disease is dry eye, keratitis, conjunctivitis, corneal erosion or corneal ulcer.

3. The therapeutic agent for the keratoconjunctival disease as claimed in claim 1 or 2, wherein a dosage form is an ophthalmic preparation.

4. The therapeutic agent for the keratoconjunctival disease as claimed in claim 3, wherein a concentration of farnesylacetic acid or a salt thereof is 0.001 to 1% (w/v).

5. A promoter of production of mucin comprising farnesylacetic acid or a salt thereof as an active ingredient.

6. A method of treating a keratoconjunctival disease comprising administering to a patient an effective amount of farnesylacetic acid or a salt thereof.

7. The method of treating the keratoconjunctival disease as claimed in claim 6, wherein the keratoconjunctival disease is dry eye, keratitis, conjunctivitis, corneal erosion or corneal ulcer.

8. A method of treating a keratoconjunctival disease comprising instilling into a patient an effective amount of farnesylacetic acid or a salt thereof.

9. The method of treating the keratoconjunctival disease as claimed in claim 8, wherein farnesylacetic acid or a salt thereof is instilled at a concentration of 0.001 to 1% (w/v).

10. A method of promoting production of mucin comprising administering to a patient an effective amount of farnesylacetic acid or a salt thereof.

11. Use of farnesylacetic acid or a salt thereof in the manufacture of a therapeutic agent for a keratoconjunctival disease.

12. The use of farnesylacetic acid or a salt thereof as claimed in claim 11, wherein the keratoconjunctival disease is dry eye, keratitis, conjunctivitis, corneal erosion or corneal ulcer.

13. The use of farnesylacetic acid or a salt thereof as claimed in claim 11 or 12, wherein a dosage form is an ophthalmic preparation.

14. The use of farnesylacetic acid or a salt thereof as claimed in claim 13, wherein a concentration of farnesylacetic acid or a salt thereof is 0.001 to 1% (w/v).

15. Use of farnesylacetic acid or a salt thereof in the manufacture of a promoter of production of mucin.
